# EUROPEAN PATENT SPECIFICATION

(11) **EP 0 649 667 B1**
(45) Date of publication and mention of the grant of the patent: **28.02.2001**
(21) Application number: 93202943.2
(22) Date of filing: 20.10.1993
(51) Int. Cl.: A61N 5/067, H01S 5/042, A61K 49/00, A61K 47/48

(54) **Quantum energy therapeutic biostimulation apparatus**
Gerät zur therapeutischen Biostimulation mittels Quantenenergie
Appareil thérapeutique de stimulation biologique à niveau d'énergie quantique

(43) Date of publication of application: 26.04.1995
(73) Proprietor: Aprile Carpenter, Antonella, Little Rock, AR 72211 (US); Carpenter, Frederick M., Little Rock, AR 72211 (US)
(72) Inventor: Aprile Carpenter, Antonella, Little Rock, AR 72211 (US); Carpenter, Frederick M., Little Rock, AR 72211 (US)
(74) Representative: Marietti, Giuseppe

(56) References cited:
- EP-A- 0 435 506
- WO-A-89/01630
- WO-A-90/15059
- WO-A-91/03276
- WO-A-91/18006
- DE-A- 3 935 257
- FR-A- 2 577 425
- US-A- 4 321 251
- US-A- 4 973 848
- SIEMENS COMPONENTS, vol.24, no.3, June 1989, MUNCHEN, DE pages 117 - 118, XP000036091 K. FISCHER 'Simple drive circuit for laser arrays using power Op-Amps'
- PATENT ABSTRACTS OF JAPAN vol. 8, no. 285 (E-287) (1722) 26 December 1984 & JP-A-59 151 481 (NIPPON DENKI) 29 August 1984
- PATENT ABSTRACTS OF JAPAN vol. 6, no. 251 (E-147) 10 December 1982 & JP-A-57 152 177 (FUJITSU) 20 September 1982
- S. BUDAVARI ET AL. (ED.): THE MERCK INDEX, 11TH EDITION, 1989 , MERCK & CO., INC. RAHWAY, N.J. US, PAGE 411 * Number 2629 *
- S. BUDAVARI ET AL. (ED.): THE MERCK INDEX, 11TH EDITION, 1989 , MERCK & CO., INC., RAHWAY, N.J. US, PAGE 1433 * Number 9041 *
- S. BUDAVARI ET AL. (ED.): THE MERCK INDEX, 11TH EDITION, 1989, PAGES 208-209 , MERCK & CO., INC. RAHWAY, N.J. US * Number 1366 *
- DRUG DESIGN AND DELIVERY, vol.1, no.3, February 1987, SWITZERLAND pages 253 - 260 M. IWATA ET AL. 'Double-Layered Stick-Type Formulation of Bleomycin for Treatment of Uterine Cervical Cancer'
- CURRENT MICROBIOLOGY, vol.25, no.2, August 1992 pages 77 - 81 M. WILSON ET AL. 'Sensitization of Oral Bacteria to Killing by Low-Power Laser Radiation'
- MEDECINE ET HYGIENE, vol.45, no.1684, 7 January 1987, SWITZERLAND pages 32 - 42 P. LAUGIER ET AL. 'Dermatologie'
- ARCHIVOS DE BRONCONEUMOLOGIA, vol.20, no.5, 1984, SPAIN pages 210 - 215 A. SANCHEZ PALACIOS ET AL. 'Participacion de Analgesicos y Colorantes en el Asma Bronquial'
- EUROPEAN JOURNAL OF RESPIRATORY DISEASES, SUPPL. NO. 126, vol.62, 1983, DENMARK pages 151 - 156 R. PANNIER 'Drug Induced Lung Disease'
- THE JOURNAL OF THE AMERICAN MEDICAL ASSOCIATION, vol.245, no.23, 1981 pages 2408 - 2410 A. MASHBERG 'Tolonium (Toluidine Bleu) Rinse - A Screening Method for Recognition of Squamous Carcinoma'

## Description

The present invention relates to an apparatus for removal of contaminants from human and animal, tissues. The apparatus is particularly useful for treatment of tumors.

In medical circles, it is well known the widely proven regression effect produced on primary and secondary tumors by hyperthermia.

Many studies, both biological and clinical, have been successfully completed, relying on localized or total-body hyperthermia.

However the success rate, although non-negligible, is absolutely unsatisfactory; the percentage of patients showing total remission, without metastases originated recurrence, is too low to qualify this particular therapy as a basis for a cure.

The reason for such a low efficacy cannot be attributed to an unpredictable mechanism, upon which the treatment is based, since heat induced cell necrosis is a well known fact, no longer debatable. The reason why hyperthermia, as cancer therapy, is only occasionally successful originates from the fact that it relies on too small a difference in irreversible-damage threshold-temperature between normal and neoplastic cells.

Such restriction is a necessary precaution for the safety of the patient, since no method nor machine exists, as at today, to deliver the required heat only to the malignant presence in the body.

The use of elevated temperature for the treatment of cancer goes back to ancient times as total-body hyperthermia, while localized hyperthermia is a product of relatively recent times, utilizing Radio Frequency or Microwave technology and even laser energy sources.

But again all of the above rely on the indiscriminate exposure of tissue to elevated temperature and are therefore restricted in scope because they must rely exclusively on the questionable and variable higher sensitivity to temperature displayed by malignant tumors.

More recently fractionated doses of Radiofrequency Hyperthermia have been proven effective, still relying on the assumed greater heat sensitivity of neoplastic growth; the procedures and results are described in Journal of Dermatol. Surg. Oncology, Vol. 15 pages 845 to 849 (1989), in an article entitled "Radiofrequency Hyperthermia Therapy of Murine Melanoma: A Comparison of Fractionated Versus Single-Dose Treatments".

However even if encouraging, still the method is clumsy and it is not backed by the existence of a commercially viable hardware system.

Although still experimental, one commercially available technique called Photodynamic Therapy appears to resemble very closely the hereby introduced method (i.e. QUETBISM), but at the same time is totally off this target and it is also missing a truly effective hardware system. However Photodynamic Therapy, like QUETBISM, is also taking advantage of the already mentioned fact that cancerous cell tend to retain chemicals and chromophores much longer than healthy tissues. On the other hand Photodynamic Therapy is relying on an alleged chemical reaction which supposedly occurs when HPD retaining tissue is exposed to light of a specific wavelength, as described in many published Research papers one of which is: "Pharmacological Modulation of Photodynamic Therapy with Hematoporphyrin Derivative and Light" appearing in "Cancer Research", Vol 47, pages 971-974 (1987). As an alternative cancer treatment this therapy method is offered to the public by QLT of Vancouver, BC.

Finally, the ability to selectively affect stained tissue, under illumination by laser light of adequate wavelength, is empirically confirmed with a research report, published in the Proceedings of the National Academy of Science, USA, Vol. 85, pages 5454-5458 (Aug 1988) and entitled: "Selective Destruction of Protein Function by Chromophore-Assisted Laser Inactivation".

FR-A-2 577 425 discloses a laser treatment apparatus comprising two distinct semiconductor laser sources (20, 40) and one gas laser generator acting as a visible light pointer. Each of the laser sources (20, 40) has a plurality of laser diodes (21-24) (41-44) that are selectively driven by circuits (11). The laser light beams of laser source 40 are grouped together into one light beam carried by optical fibres; the laser beams of source 20 are directly irradiated from the laser diodes 21-24.

First objective is to provide an apparatus suitable for carrying out a self sufficient non-synergetic technique for the effective obliteration of unwanted extraneous presence in the human or animal tissues, tumor growth or viral contaminants that they might be; such therapy is in fact called Enhanced Hyperthermia. With "tissues" is here meant any aggregation of cells besides body tissues, i.e. also blood. The apparatus comprises an opto-electronic hardware (LAILT-System).

A second objective is to provide the subsystem capable to deliver the required energy to any location of interest within the body, for the treatment of malignant growths.

A third objective is to provide the modification to the main hardware system required to transform it from a Cancer Treatment Device to a Blood Viral Contaminants Treatment Device; namely: the variations between the LAILT System I and II.

The final objective is to establish guidelines on the type and characteristics of the family of non-toxic compounds which may be used to correctly implement the Enhanced Hyperthermia Treatment.

The present invention provides an apparatus for selectively removing cellular or viral contaminants from human or animal tissues according to claim 1.

Moreover, the invention relates to a new use of tagging compounds for cancer cells according to claim 11. The invention being discussed has taken all the positive aspect of recent research efforts and presently used techniques and has given life to the method according to present invention, i.e. Enhanced Hyperthermia, which combines the guiding principles of most of the existing methods with the simple Quantum Physics' principle of Blackbody radiation.

Selective targeting of only malignant tissue is achieved through the widely utilized standard method of neoplastic tissue tagging, followed by illumination with Quantum Energy, namely laser light of a wavelength not readily absorbed by live tissue, under normal circumstances. Requirement on the tagging agent are: non-toxicity to human cells and a spectrum of absorption with a maximum in the frequency range of the light source utilized.

Main feature, that gives the invention a practical general use, is the invention apparatus, i.e. the LAILT System, which can deliver laser light to any desired physical location and selectively produce irreversible damage to cell protein, without causing charring or secondary damage, on account of design features which render the power output controllable and uniformly distributed.

The invention will now be disclosed in further detail with reference to accompanying and non limiting drawings, where:
- FIG.1 is an elementary visualization of the mechanism of light absorption by tissues with different pigmentation.
- FIG. 2 is a diagram visualization of an embodiment of the invention, with a symbolic number of array sources for monochromatic light.
- FIG. 3 is a schematic representation of a preferred embodiment of an individual channel's driver circuit.
- FIG. 4-7 are a schematic representation of a preferred peripheral attachment for proper delivery.
- FIG. 8 is a diagram of a modified internal layout, required for a different application of the invention.

From the medical point of view, the invention takes into account what has been done to this point in the treatment of cancer, combines several partially successful approved techniques and brings the entire ensemble a step forward, above and beyond what each and every method was intended or has been interpreted to be doing, and so arrives at a successful solution which has a full theoretical explanation.

From the technology point of view, the invention is taking semi off-the-shelf opto-electronic components and is utilizing them in a manner different enough from standard procedures to create new and inventive method (QUETBISM) and apparatus.

As already pointed out QUETBISM represents the method for delivery of an improved form of hyperthermia, identified by the inventors with the name of "Enhanced Hyperthermia".

Based on sound and already proven principles of physics and biology, QUETBISM produces Enhanced Hyperthermia within a specific target, through the utilization of one of the LAILT Systems.

The actual bio-mechanism requires the intra venous injection of a chomophore carrying compound, which is non-toxic and does not causes any particular chemical reaction within the tissue, but simply delivers the stain to the general location.

Based on an important finding already widely exployted in many other cancer treatments, after a waiting period of 36 to 48 hours, healthy cell will have eliminated the compound from their structure, while the sick cells will not have. Therefore they will maintain a different index of absorption, at least for light of the selected wave length.

The LAILT apparatus will then be used to illuminate the general area and, since its wavelength was specifically selected to be in a range of negligible absorption by normal tissue, only the abnormal cells 3, retaining the stain, will reach the target temperature of 50 degrees Celsius.

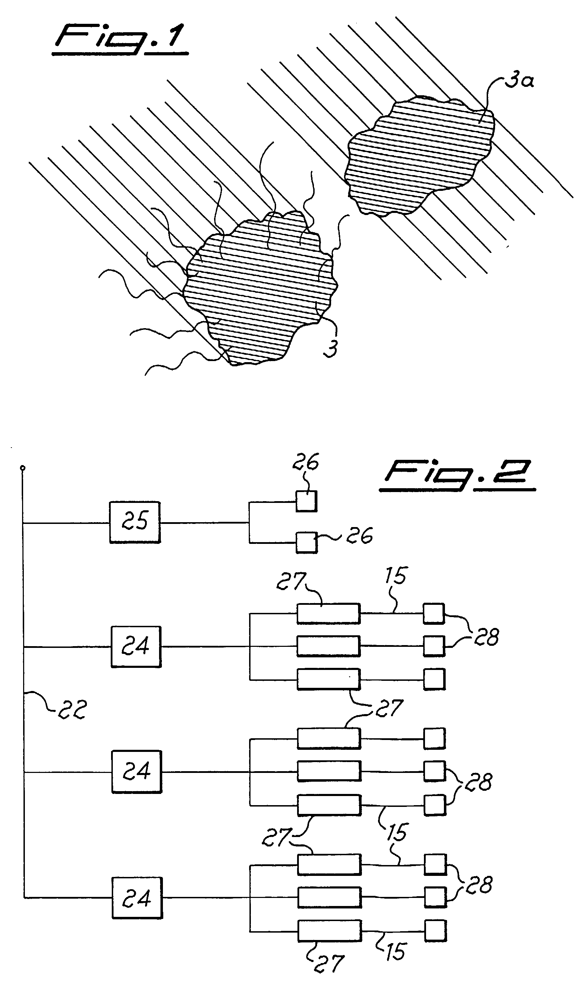

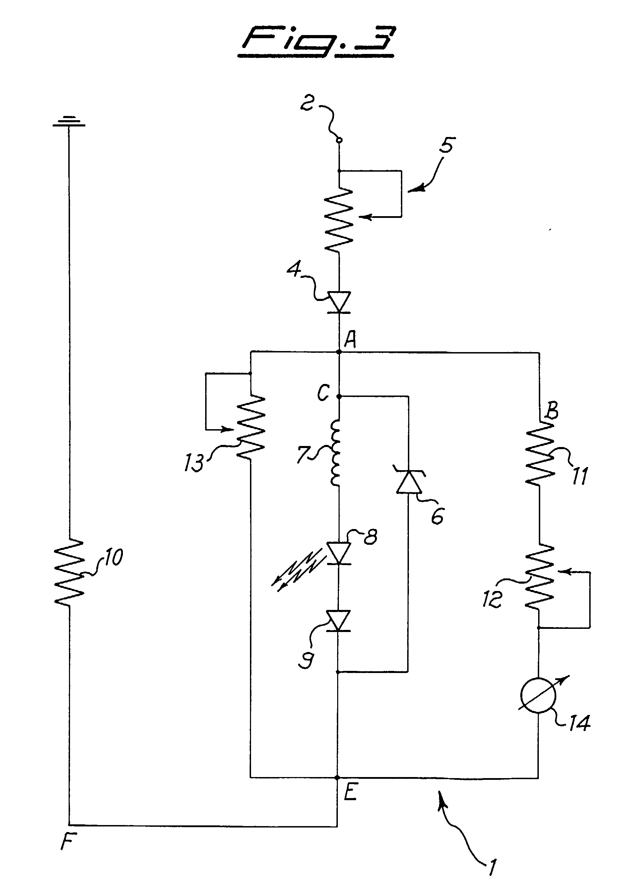

At such temperature cell necrosis is certain; however the surrounding healthy cells 3a, now free of stain, will not be able to reach a temperature greater that 42-43 degrees Celsius, which is still within a safe margin for survival, especially for normal cells (fig. 1).

For these reasons, this innovative treatment will not require the assistance of imaging devices, nor it will require the incorporation of temperature monitoring instruments.

The LAILT apparatus is designed to safely deliver, to the general surroundings of a targeted area, a controlled amount of energy low enough to cause only minor discomfort and totally reversible damage, if any, to healthy tissue but sufficient to produce irreversible damage to regions displaying abnormal index of absorption, artificially induced with the tagging process.

This goal cannot be achieved with the assistance of any traditional laser system, presently used in medicine.

The LAILT System I is so compact that it may be contained in a metal casing with the approximate size of 47x31x18 cm and may be carried in a compact suitcase or a slightly oversized attache; it is designed (fig. 2) to plug directly on an ordinary 120 (or 220) Volt standard wall outlet and does not require an actual cooling system. The electrical cord 22 coming from the wall outlet 23 feeds both a series of independent power supplies 24 and a DC transformer 25. The transformer feeds two mini fans 26, which represent the only cooling apparatus needed. Each power supply feeds the driver circuits of three channels 27; where the word channels is here used to identify the laser energy sources.

Consequently the actual number of power supplies depends solely on the number of channels used in the system, which in turn depends on the specific application.

The energy sources in question are in fact semiconductor lasers of high output and uniform, unfocused far field pattern, with an output rating of at least 200 mW at the window or fiber tip, depending on the application.

For the LAILT System I, the semiconductor lasers utilized, also called laser diodes because of their design layout, have a fiberoptic line 15 approximately one (1) meter long or thereabout attached at the emission window; the rated output at the laser light emission sources 28, i.e. the tips of the fibers, is in the general range of 150-300 mW.

However for the specific need of the Enhanced Hyperthermia treatment, the devices are not driven to their maximum rating since, for curative purposes, it is not necessary to induce tissue charring.

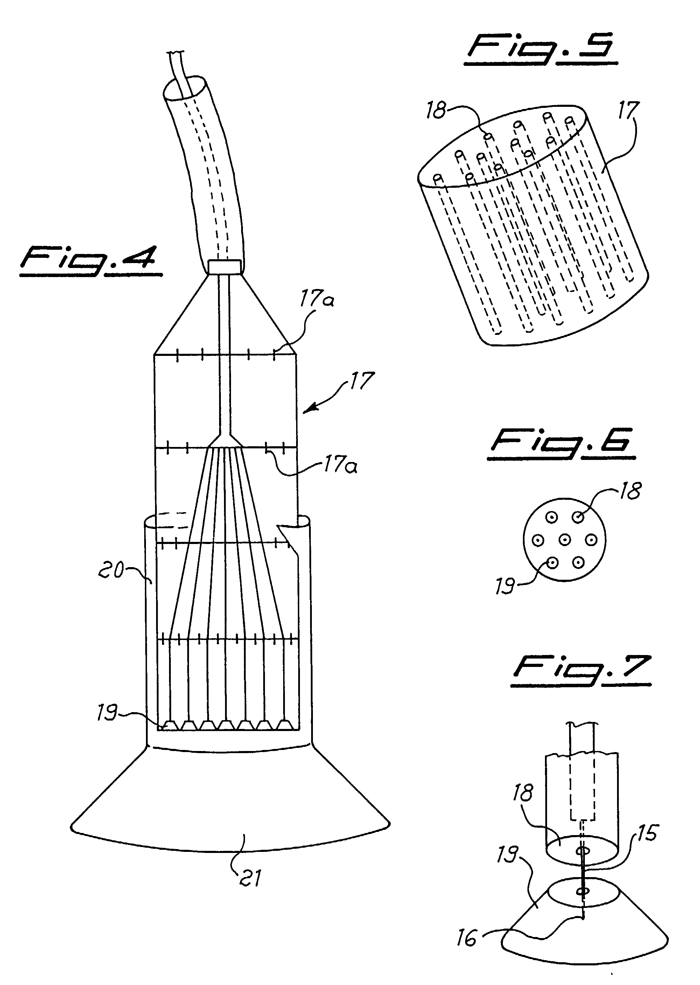

Each laser is independently powered by a separate driver circuit 1, (fig. 3), to take into account and be protected from individual minor variation in rating; so that each channel can deliver on tissue an equal amount of power and the combined output beam can furnish to the illumination area a uniform amount of energy.

Each driver circuit is also provided with safety oriented filtering features, to eliminate the costly possibility of damage to the very sensitive semiconductor lasers.

Said circuits are also equipped with an input power control device (not shown), manually operated and accessible from a numbered nub on the external top portion of the casing, below each numbered meter, in turn intended to provide visual confirmation of the exact amount of input provided to each and every energy source.

The identical driver circuits 1 are at this point very simple, efficient and to the point. At a later date, they will eventually be improved to render the LAILT System more user friendly; but the basic structure will remain unchanged because it is capable to guarantee safe and controlled operation of the system. More specifically, the basic circuitry comprises : control means 4,5 to control the voltage drop from power source to semiconductor laser; protecting means 6,7 to protect said semiconductor laser 8 from current surges and voltage spikes; filtering means to protect the semiconductor laser from ground surges and spikes; and control means 10-13 to control biasing of semiconductor laser.

As it is clearly comprehensible from the schematic in FIG. 3, the input power from 2 is controlled with variable resistor 5 followed by diode 4, whose presence only serves to ensure finely adjusted voltage drop.

At point A the line separates into three parallel resistor networks, in this way effectively dividing the total current into branches of appropriate level. This is done to provide the semiconductor laser 8 with correct biasing and a variable drive current, which in turn permits operation of said laser throughout its full range of output capability.

The branch including connection point C is the one containing the semiconductor laser and, for reasons already indicated, it is designed to see a current flow variable from the lasing threshold of approximately 175 mAmps to the maximum allowable of approximately 800 mAmps.

The branch containing connection point B includes one fixed resistor 11 and a variable one 12, in addition to one Amp meter 14.

The presence of this parallel branch serves the double purpose of providing an adjustable current shunt for the semiconductor laser as well as an indirect monitor of the diode's drive current.

Fixed resistor 11 establishes the minimum conductance (G) of this branch, while variable resistor 12 allows for adjustments. An increase in the value of 12 causes an increase in the laser's drive current, while a decrease in the value of the same resistor produces a reduction in laser light output power.

The meter 14 following 12 allows the trained user to monitor the channel, by determining the semiconductor laser's drive current through a simple subtraction of the value indicated by the meter from the known total current.

In the branch containing the laser, coil 7 is incorporated to delay any eventual surge current, until the Zener Diode 6 has the opportunity to turn on and divert from the very sensitive light source 8 any voltage spike.

Additionally diode 9 is located on the cathode side of the semiconductor laser to filter out ground surges and spikes.

Variable resistor 13 is incorporated to allow for finer tuning of current adjustments in both B and C branches.

The three parallel branches rejoin at point E, proceed to include point F in their path and finally flow through fixed resistor 10, which in conjuntion with the total resistence of of the three branch circuit helps establish proper loading of the power supply.

To allow proper monitoring of each branch from the external side of the casing and only as an additional precaution, two LED are added to circuit with relative biasing resistors to merely confirm that current is actually flowing through the whole circuit and the branch containing the laser. No illuminating signal is necessary in the third branch, since the meter itself is the utilized indicator.

Finally and not shown in any of the drawings, for the LAILT System I only the Quantum Energy emerges from the tip of a fiberoptic line installed as part of the Semiconductor laser at the original manufacturer's site.

The central feature, without which the proper delivery of Enhanced Hyperthermia would not be possible, and, at the same time one of several technological innovations in the LAILT System, is the presence of multiple fiberoptic guided beams of nearly identical power, originating from distinct individually driven laser energy sources, which ensure uniform distribution of power within the illuminated region.

The latter characteristic is vital for the certain acquisition of the target temperature (50 degrees Celsius) by tagged tissue only, as the only way to ensure its total necrosis.

Systems equipped with multiple fiberoptic lines, utilizing the electromagnetic energy originating from one single powerful laser source, cannot allow for proper control of the output power.

In other words, the present invention apparatus provides also a method for the generation of uniform and unfocused monochromatic laser light and irradiation by the same of an extended area.

The other key factor is the utilization of semiconductor laser technology, which not only provide monochromatic radiation, in the exact range not significantly absorbed by human tissue under normal circumstances, but it also renders possible the design of an "Array of Arrays", namely the LAILT System, which, even as a system of multiple laser sources, displays very limited input power consumption, and it is easily transportable, practical, does not require a clumsy cooling apparatus and is still of reasonable cost, contrary to what a system of multiple traditional lasers would be.

In the preferred embodiment the two fans 26 are strategically positioned, with respect to driver circuits, to provide spread and uniform ventilation of the array of said circuits 27. Each laser source is firmly resting on the already mentioned angled heat sinks, and as a whole are positioned in apparent disorderly fashion; in reality the distribution has been so designed to ensure that there is a reasonably close distance covered and a minimum overlapping in the fiber path, as they come together in a bundle and are so directed toward the exit opening positioned to the side of the casing. This distribution is the most adequate and consistent with the need to limit the spacial demands. Finally, at the exit point in the casing, the fiber bundle is firmly braced to the internal side of the apparatus external packaging, for the sole purpose of limiting the stress, deriving from motion in repeated usage, on the fiber optic attachement located for each laser source at the window of emission.

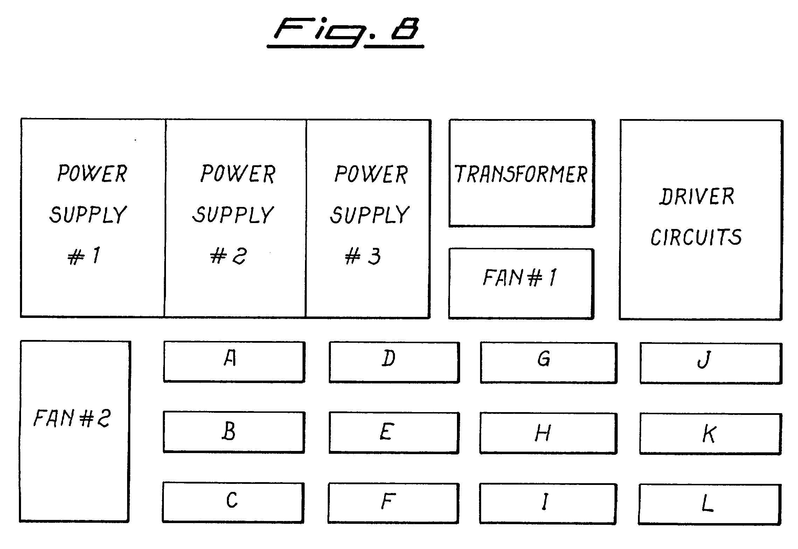

In the LAILT apparatus to be used for the treatment of melanoma the delivery attachment will be as represented in FIG. 4 to 7, where a plexiglass tube 17 is used to guide, separate and maintain securely in place the various fiber lines 15.

This goal is achieved with a multi sections (three to five or possibly more) solid acrylic tube 17, where channels 18 have been etched for the passage of the fibers 15 in a smoothly progressive diverging fashion at first, until appropriate separation has been achieved and subsequently in a collimated pattern until the exit opening 19 is reached; at said opening the tip 16 of the optical fiber protrudes from the channel and is kept away from direct contact with the tube by means of an enlarged cone shaped channel end 19, and from the surface of the skin by a simple base collar cladding 20 doubling also as stand thanks to a cone shaped end portion 21. All the above features in the design of the delivery attachment are necessary precautions not only for the convenient handling of the tool, but especially to ensure that the tips of the various fiber lines do not come in direct contact with each other or the surface of the skin and do not in this way cause self damage nor generate unnecessary overheating of the target.

The total length of the delivery tubule is approximately 13 cm and the various sections snap into place through the use of incorporated pins 17a.

Finally the tagging agent necessary to alter the index of absorption of the neoplastic tissue is not a uniquely identified nor an exclusive product, since no specific or obscure chemical reaction are at the basis of the process.

Again all is required of any adequate product is to be capable to carry an energy transferring compound, that is preferably a chemically inert and pharmaceutically acceptable chromophore, to the desired site, which does not need precise spacial identification, but merely an approximate one simply pointing to the general region.

Restrictions on the chromophore are: non toxicity to the human body and most preferably a shade of eighter green or black coloration. In the studies reported in the previously mentioned article discussing chromophore-assisted laser inactivation and during that particular research effort, Malachite Green was used as stain in question, but, although within the acceptable chromatic range, malachite green is not proven to be totally non-toxic to the body.

In vitro tests were carried out according to the following example.

### Example

In vitro tests were effected according to the following procedure.

About 0.5 cc of Agar gel were stained with a 5%concentration of McCornic Food Coloring containing FD&C Yellow # 5 and FD&C Blue #1. The stained Agar gel was exposed to illumination from the invention apparatus, utilized with an output of 200 milliWatts, from a 1.5 cm distance for 90 seconds time interval and longer. A thermocouple tip immersed in the gel was used for immediate and correct temperature read-out.

An equal volume of unstained Agar gel was also subjected to identical condition of exposure.

The results showed that stained Agar gel did reach a 50 °C temperature within the initial 90 seconds, while non stained agar gel did not go above the 43 °C mark, even well beyond the 90 sec. time limit.

In the actual treatment of patients it will preferably be used a more convenient compound, like one of the stain already used for the visualization of cancer; one of which is the one identified by the name of "Fresh Green".

Similarly, it will also be preferred the use of some of the products which display affinity to highly acid tissue as cancer and are used to quickly identify the neoplastic site, without the need of a waiting period.

According to another preferred embodiment of the invention, these products will be admixed with a color in the proper range, to actually shorten the waiting period prior to illumination treatment.

The same principle and technique may be applied in the elimination of viral contaminant in the blood, although a very different delivery apparatus will be needed in such a case.

Again, in this different application, the main structure of the LAILT System remains unaltered and so do the various driver circuits; only the internal arrangement of the "Array of Arrays" is modified, as shown in the black box diagram of FIG. 8. The differences from the previously disclosed embodiment are that laser sources A to L do not need to be oriented in a distance balancing fashion, since slightly different semiconductor lasers are utilized in this design and therefore it is only necessary to provide uniform illumination of the target. The difference in these laser sources only applies to the external packaging and it refers to the fact that they do not carry a fiberoptic attachment and relative fiber, but they simply possess an emission window from where the beam irradiate with a far field pattern of slightly oval cross-section instead of the circular one of the output from the fiberoptics. However, in this case the exiting beam retains the coherency characteristic of lasers, which is lost in the case of devices provided with fiberoptic lines. Finally, the sources will be resting on ordinary heat sinks with emission windows facing upward, immediately below a clear window area in the apparatus casing.

## Claims

1. A laser apparatus for selectively removing cellular or viral contaminants from human or animal tissues by hyperthermia therapy comprising a plurality of distinct semiconductor lasers (8) and a plurality of light delivery means (15), said semiconductor lasers (8) being laser diodes for generating laser-light having wavelength that is not significantly absorbed by healthy tissues (3a) but that is absorbed by said contaminants (3) when stained with energy transferring compounds, each of said laser diodes being connected to and independently powered by separate driver circuits (1); said light delivery means (15) comprising a plurality of optical fibers (15), each one of said optical fibers (15) being connected to the light output of one of said laser diodes (8), thereby providing multiple fiberoptic guided beams originating from distinct individually driven laser diodes for uniformly irradiating an area of said tissues with said laser light to have said energy transferring compounds release energy to stained contaminants to heat them to a temperature resulting in their irreversible damage.

2. An apparatus according to claim 1, wherein said driver circuit further comprises : control means (4,5) to control the voltage drop from power source to laser diode; protecting means (6,7) to protect said laser diode(8) from current surges and voltage spikes; filtering means to protect the semiconductor laser from ground surges.

3. An apparatus according to claim 2, wherein said control means for controlling voltage drop comprises a variable resistor (5) and optionally a diode (4), said protecting means comprises an impedance coil (7) and a zener diode (6), said filtering means comprises a diode (9), and said biasing control means comprises one or more fixed resistors (10,11) and one or more variable resistors. (12,13).

4. An apparatus according to claims 2 or 3, further comprising an ammeter (14) or similar drive current monitoring means.

5. An apparatus according to claims 1 to 4, further comprising housing means to individually house and separate from each other the tips (16) of said optical fibers and their end portions.

6. An apparatus according to claim 5, wherein said housing means comprises a tube (17) having a plurality of channels (18) each housing one optical fiber (15), each channel having a cone-shaped end portion (19) housing said fiber tip (16) without contacting it and in spaced relationship with respect to the patient skin.

7. An apparatus according to claim 6 wherein said tube (17) is provided with a cladding (20) having cone-shaped end portion (21).

8. An apparatus according to claims 6 or 7, wherein said tube (17) comprises a plurality of sections.

9. An apparatus according to any previous claim, wherein said laser light has an output within the range from 100 to 300 mw.

10. Use of tagging compounds for cancer cells in combination with pharmaceutically acceptable chromophores for the manufacturing of energy transfer compositions for hyperthermia therapy by irradiation with laser light generated by an apparatus according to any claim 1 to 9.

## Patentansprüche

1. Laservorrichtung zur selektiven Entfernung zellulärer oder viraler Kontaminationen von menschlichen oder tierischen Geweben mittels eines Hyperthermieverfahrens, umfassend eine Vielzahl von verschiedenen Halbleiterlasem (8) und eine Vielzahl von Lichtabgabemitteln (15), wobei die Halbleiterlaser (8) Laserdioden zur Erzeugung von Laserlicht besitzen und das Laserlicht eine Wellenlänge besitzt, die nicht signifikant von gesunden Geweben (3a) absorbiert wird, die aber von den Kontaminationen (3) absorbiert wird, wenn diese mit Verbindungen zur Energieübertragung gefärbt werden, wobei jede dieser Laserdioden mit einem separaten Stromkreis (1) verbunden ist und unabhängig von diesem mit Energie versorgt wird; und diese Lichtabgabemittel (15) eine Vielzahl optischer Fasern (15) umfassen, wobei jede dieser optischen Fasern (15) mit einem Lichtauslass einer der Laserdioden (8) verbunden ist und dadurch eine Vielzahl faseroptisch geführter Lichtstrahlen ausgehend von verschiedenen individuell betriebenen Laserdioden zur Verfügung gestellt werden zur gleichmäßigen Bestrahlung eines Bereiches dieses Gewebes mit diesem Laserlicht, so dass die Energie übertragende Verbindungen Energie an die gefärbten Kontaminationen abführen und diese auf eine Temperatur aufheizen, bei der eine unumkehrbare Zerstörung der Kontaminationen erfolgt.

2. Vorrichtung nach Anspruch 1, wobei der Stromkreis folgendes umfasst: Regelungsmittel (4, 5), um die Spannungsverringerung von der Spannungsquelle zur Laserdiode zu regeln; Schutzmittel (6, 7), um die Laserdiode (8) von Stromstärkestößen und Spannungsspitzen zu schützen; Filtermittel um die Halbleiterlaser von Erdungsstromstößen.

3. Vorrichtung nach Anspruch 2, wobei die Regelungsmittel zur Regelung der Spannungsverringerung einen variablen Widerstand (5) und wahlweise eine Diode (4) umfassen, wobei die Schutzmittel eine lmpedanzspule (7) und eine Zener-Diode (6) umfassen, wobei die Filtermittel eine Diode (9) umfassen, und wobei die Regelungsmittel für die Biasspannung eine oder mehrere feste Widerstände (10, 11) und eine oder mehrere variable Widerstände (12, 13) umfassen.

4. Vorrichtung nach den Ansprüchen 2 oder 3, weiter umfassend ein Amperemeter (14) oder ähnliche Mittel zur Anzeige der Stromstärke.

5. Vorrichtung gemäß Ansprüchen 1 bis 4, weiter umfassend Gehäusemittel, um die Spitzen (16) der optischen Fasern und ihre Endbereiche einzeln aufzunehmen und voneinander zu trennen.

6. Vorrichtung nach Anspruch 5, wobei die Gehäusemittel eine Röhre (17) umfassen, die eine Vielzahl von Kanälen (18) besitzt, wobei jeder Kanal eine optische Faser (15) aufnimmt, und wobei jeder Kanal einen konisch geformten Endbereich (19) besitzt, der die Faserspitze (16) aufnimmt, ohne diese zu berühren, und die Faserspitze (16), bezogen auf die Haut des Patienten, auf Abstand gehalten wird.

7. Vorrichtung nach Anspruch 6, wobei die Röhre (17) mit einer Hülle (20) ausgestattet ist, die einen konisch geformten Endbereich (21) aufweist.

8. Vorrichtung nach den Ansprüchen 6 oder 7, wobei die Röhre (17) eine Vielzahl von Sektionen umfasst.

9. Vorrichtung nach einem der vorigen Ansprüche, wobei das Laserlicht eine Ausgangsleistung im Bereich von 100 bis 300 mW besitzt.

10. Verwendung von Verbindungen zur Markierung von Krebszellen in Verbindung mit pharmazeutisch akzeptablen Chromophoren zur Herstellung von Verbindungen zur Energieübertragung für eine hyperthermische Therapie durch Bestrahlung mit Laserlicht, das mit einer Vorrichtung gemäß einem der Ansprüche 1 bis 9 erzeugt wird.

## Revendications

1. Appareil laser destiné à éliminer sélectivement des contaminants cellulaires ou viraux de tissus humains ou animaux par thérapie hyperthermique, comprenant une pluralité de lasers à semi-conducteur (8) distincts et une pluralité de moyens de fourniture de lumière (15), lesdits lasers à semi-conducteur (8) étant des diodes laser pour émettre une lumière laser ayant une longueur d'onde qui n'est pas absorbée, de manière significative, par des tissus sains (3a) mais qui est absorbée par lesdits contaminants (3) lorsqu'ils sont marqués ou colorés par les composés de transfert d'énergie, chacune desdites diodes laser étant reliée à des circuits d'attaque distincts et alimentée de façon indépendante par ces derniers (1) ; lesdits moyens de fourniture de lumière (15) comprenant une pluralité de fibres optiques (15), chacune desdites fibres optiques (16) étant reliée à la sortie de lumière d'une desdites diodes laser (8), définissant, de ce fait, de multiples faisceaux guidés par fibre optique prenant leur origine à partir de diodes laser distinctes attaquées de façon individuelle pour exposer à ladite lumière laser, de manière uniforme, une zone desdits tissus, de telle manière que lesdits composés de transfert d'énergie libèrent de l'énergie vers les contaminants marqués ou colorés afin de les chauffer jusqu'à une température aboutissant à leur détérioration irréversible.

2. Appareil selon la revendication 1, dans lequel ledit circuit d'attaque comprend, de plus : des moyens de commande (4, 5) destinés à agir sur la chute de tension de la source d'énergie électrique à la diode laser ; des moyens de protection (6, 7) servant à protéger ladite diode laser (8) contre des surintensités et des pointes de tension ; des moyens de filtrage destinés à protéger le laser à semi-conducteur contre des pointes de tension à la masse.

3. Appareil selon la revendication 2, dans lequel lesdits moyens de commande destinés à agir sur la chute de tension comprennent une résistance variable (5) et, si on le désire, une diode (4), lesdits moyens de protection comprennent une bobine d'arrêt (7) et une diode Zener (6), lesdits moyens de filtrage comprennent une diode (9), et lesdits moyens de commande de polarisation comprennent une ou plusieurs résistance(s) fixe(s) (10, 11) et une ou plusieurs résistance(s) variable(s) (12, 13).

4. Appareil selon la revendication 2 ou 3, comprenant en outre un ampèremètre (14) ou un moyen similaire de surveillance du courant d'attaque.

5. Appareil selon les revendications 1 à 4, comprenant en outre un moyen formant logement pour recevoir individuellement et séparer les uns des autres les bouts (16) desdites fibres optiques et leurs parties d'extrémité.

6. Appareil selon la revendication 5, dans lequel ledit moyen formant logement comprend un tube (17) ayant une pluralité de conduits (18) logeant chacun une seule fibre optique (15), chaque conduit présentant une partie d'extrémité de forme conique (19) recevant ledit bout de fibre (16) sans le toucher et avec un espace par rapport à la peau du patient.

7. Appareil selon la revendication 6, dans lequel ledit tube (17) est pourvu d'un gainage (20) comprenant une partie d'extrémité de forme conique (21).

8. Appareil selon la revendication 6 ou 7, dans lequel ledit tube (17) comprend une pluralité de sections.

9. Appareil selon l'une quelconque des revendications précédentes, dans lequel ladite lumière laser a une puissance de sortie comprise dans la plage de 100 à 300 mW.

10. Utilisation de composés de marquage pour des cellules cancéreuses en combinaison avec des chromophores acceptables sur le plan pharmaceutique pour la fabrication de compositions de transfert d'énergie pour une thérapie hyperthermique par exposition à une lumière laser produite par un dispositif selon l'une quelconque des revendications 1 à 9.
